Europäisches Patentamt

European Patent Office

Office européen des brevets

(11) Publication number: **0 252 044**
A1

## (12) EUROPEAN PATENT APPLICATION

(21) Application number: **87850190.7**

(22) Date of filing: **11.06.87**

(51) Int. Cl.⁴: **A 61 K 49/00**
**A 61 B 10/00, A 61 M 35/00**

(30) Priority: **26.06.86 SE 8602841**

(43) Date of publication of application:
**07.01.88 Bulletin 88/01**

(84) Designated Contracting States:
**AT BE CH DE ES FR GB GR IT LI LU NL SE**

(71) Applicant: **PHARMACIA AB**
**Rapsgatan 7**
**S-751 82 Uppsala (SE)**

(72) Inventor: **Resul, Bahram**
**Vitkalsgatan 112**
**S-754 49 Uppsala (SE)**

(54) **Test strip and method for epicutaneous testing.**

(57) Test strip having at least one patch thereon and intended for use in occlusive epicutaneous testing, characterized in that on at least one of the patches the test substance is present as an inclusion complex with a cyclo compound capable of forming inclusion complexes with the test substance. A method for epicutaneous testing utilizing the test strip is also covered.

EP 0 252 044 A1

Bundesdruckerei Berlin

**Description**

## TEST STRIP AND METHOD FOR EPICUTANEOUS TESTING

Technical background

This invention relates to occlusive epicutaneous testing ("patch tests"). In particular, the invention is concerned with test strips carrying at least one patch that contains a test substance.

Epicutaneous testing is carried out for determining whether or not a patient suffers from contact sensitization to specific substances (allergens) or for testing allergenic and/or irritant properties of a substance. Normally the contact allergens are low molecular weight compounds. Most of them have molecular weights of less than 500 dalton, some of them 500 - 1000 dalton and there are a few above 1000 dalton. There are no important contact allergens known having molecular weights above 5000 dalton.

The term "patch test" derives from the fact that from the end of the 19th century onwards patches of fabric or paper were employed which were soaked with test substances. Many different types of patches have then been employed through the years. See for example US-A-3,703,809, US-A-3,894,531, US-A-4,214,592, US-A-4,158,359, US-A-4,390,027, US-A-4,450,844, and WO-A-8601994. In its present context the term "patch" is used in a very broad sense nowadays; in our specification and claims this term denotes the area that carries the test substance and is applied to the skin.

In the occlusive epicutaneous testing procedure, the substance suspected to have allergenic and/or irritant properties is applied to normal skin under occlusion for a certain period of time, in a correct manner and with the substance incorporated in a suitable formulation. This will then, in contact sensitization cases, produce an allergic eczema in the test area. Irritant substances give rise to irritative eczema reactions of a similar character (Manual of Contact Dermatitis; Fregert S, 2nd ed. Munksgaard, Copenhagen (1981), pp. 71 - 76). Sometimes irritant reactions may disturb the reading when the testing is performed in order to detect a contact allergy. Usually, the test substance is applied in a suitable vehicle, as for instance petrolatum or water. Various different types of patches are at present commercially available, the most popular ones being small cups of aluminum or plastics (Finn Chambers® from Epicon Oy, Finland, and Square-test from Chemotechnique Diagnostic AB, Malmö, Sweden, respectively), or small discs of aluminum provided with filter paper (Al-test from Holister Stier, USA). Immediately before the test is to be performed a suitably formulated test substance is applied onto the "patch" whereupon it is directly applied under occlusion to normal skin. If the vehicle is water the formulated test substance for practical reasons may be soaked into a patch of fabric or paper that has been inserted into the cup. Usually, a plurality of "patches" are arranged on an adhesive strip and covered with a detachable protective foil which is removed immediately before applying the test substances to the patches.

According to many dermatologists the new epicutaneous testing technique as presented in WO-A-8601994 and Br J Dermatol 112 (1985), pp 63 - 68, has meant a revolution within the field of epicutaneous testing. This technique employs a patch on which the test substance is preformulated in a polymeric film which will swell to form a gel when the patch is applied occlusively against the skin. The method has given very reproducible test results with considerably lesser amounts of test substance as compared to the generally accepted method that utilizes Finn Chambers®. In connection with Applicant's development of a product according to WO-A-8601994 it has been found that long-term storage of testing materials may give rise to problems in the case of some of the test substances. These had been known already previously to be problematic, because of their instability and/or relatively high vapor pressure. Examples of these substances are formaldehyde, fragrancy substances in perfumes, flavoring substances etc.

As indicated above, patch epicutaneous testing has been in use for almost a century. The practice of dispensing the test substances on the patches at the very moment when the test is about to be performed has continued all the time in ordinary clinical routine work for the reason that highquality patches carrying predispensed test substance have not been available commercially. The quality has been quite unsatisfactory. It turned out to be difficult to manufacture patches capable of holding equivalent amounts of test substances when stored for a prolonged period of time. The problem is dealt with in some of the publications mentioned above, but what they say is only that patches carrying predispensed test substances have to be stored in tightly sealed packages; leakage both from within and from the outside is to be prevented in this manner.

One problem encountered for certain critical contact allergens is the proximity between the optimal dose for allergy testing and the treshold of irritancy. This problem can be severe, results in difficulties in reading the test result and can be found in classical ways of epicutaneous testing as well as in the method of WO-A-8601994.

For a review on patch testing in allergic contact dermatitis see Fischer T and Maibach HI (Seminars in Dermatology 5 (1986) pp 214-24).

During the priority year the Swedish Patent Office has performed an International Type Search. In their report no publications were cited as novelty bars. Chemical Abstracts 100 (1984) 91355 s and SE-B-443,914 (corresponds to WO-A-8601994) were cited as being of particular relevance. Chemical Abstracts 100 (1984) 91355 s deals with the administration of cyklodextrin-steroid complexes to skin in order to achieve a therapeutic effect. This is quite a different technical field compared to that of the present invention, and the effect contemplated is also quite different from the allergic or irritant reaction required in epicutaneous testing. SE-B-443,914 is referred to in this specification as WO-A-8601994. US-A-4,214,592, US-A-3,703,890, US-A-3,515,126, US-A-3,699,963, US-A-3,734,097, GB-A-1,459,262 and Acta Pharma Suec 21 (1984) pp 357-64 were cited as defining the general state of the art to which the invention belongs.

Objects

It is an object of the invention to provide patches and test strips containing stabilized formulations of test substances which are to be employed for epicutaneous testing. Another object is the stabilization of test substances that are unstable in some way or other when formulated for epicutaneous testing on a patch. A further object is to offer methods and means according to which the influence of irritant reactions on the reading of an allergic skin reaction can be reduced. This latter object may lead to more clear-cut skin-reactions facilitating the reading of a test result. Among more specific objects may be mentioned means and methods to be employed in testing for contact sensitization and contact eczemas against formaldehyde, perfume components, spices, lanolin, fatty acids, mercapto mix and the like.

The invention

The difficulties mentioned above may be overcome by means of the present invention; at the same time the aforesaid objects can be attained to a large extent. One aspect of the invention is a test strip for occlusive epicutaneous testing.

The strip has at least one patch containing test substance. The characterizing feature of the strip is that at least one of its test substances is formulated as an inclusion complex of the substance and a cyclo compound (I) capable of forming such complexes. This type of complexes are well known to those skilled in the art and are called either "inclusion complexes" or "host-guest compounds". The cyclo compounds (I) contemplated have to be physiologically acceptable (in the first place non-allergenic and non-irritant) and pharmaceutically acceptable (that is, they must lend themselves to formulating in a desired manner for epicutaneous testing).

The most important type of compound (I) which may be of interest in this context is a polysaccharide type substance composed of suitable monosaccharide units, these units forming cyclized polysaccharide chains via glycosidic linkages.

The best known of these compounds, and most preferred according to the present invention, are the cyclodextrins. It is known that cyclodextrins may comprise from 6 up to 12 monosaccharide units, and moreover their ability to form inclusion complexes has been known for a long time; but industrially this fact has been utilized during only the last ten years or so. The most important purposes for which these compounds have been used comprise stabilization of readily oxidizable substances and of substances having a relatively strong tendency to vaporize. Such types of stabilization have been performed within domains of food industry, perfume manufacturing industry, and pharmaceutical industry. For economic reasons it has been preferred to use cyclodextrins composed of from 6 to 8 alpha-1,4-D-glucopyranose units (called alpha-, beta- and gamma-cyclodextrins respectively). For the same reason these cyclodextrins are preferred in the context of the present invention. It is a known fact that cyclodextrins can be derivatized without losing their complex-binding properties. Examples are for instance partially methylated cyclodextrin and cyclodextrin crosslinked in a manner similar to adsorbents based on dextran (Sephadex® (Pharmacia AB, Sweden) is such an adsorbent). The invention comprises also the use of complex-binding cyclodextrin derivatives in epicutaneous testing procedures. For a review of various inclusion complexes, with special emphasis on cyclodextrin and its use, see Starch/Die Stärke 34 (1982), pp. 379-85, and Die Stärke 29 (1977), pp. 26-33.

The finding that the invention gives test results that are easier to read was not expected and appeared to Applicant and the inventor as a surprise.

Further aspects of the invention will become apparent in the course of the following description and attached claims.

Test substances

A test substance when subjected to forming an inclusion complex is thereby stabilized against evaporation and/or oxidation reactions and/or reduction reactions. For some test substances stabilization is of the utmost importance; this applies in particular to substances having a vapor pressure such that significant amounts will evaporate if the substances are formulated in accordance with ordinary techniques for epicutaneous testing. Most of the problematic test substances are to be found among those having a vapor pressure exceeding 0.01 mm Hg at room temperature. Other substances that may require stabilization according to the invention are those that are prone to undergo oxidation and/or reduction. Many of the problematic substances will as a rule contain at least one functional group selected from among olefinic double bonds, primary alcoholic groups, aldehyde groups and amine groups.

The test substances which may be stabilized according to the invention may be hydrophobic or hydrophilic in character, but the greatest advantages are obtained with amphiphilic substances. Strongly hydrophilic substances may be reversibly derivatized to form a derivative of a character suitable for stabilization according to the present invention. Most of the test substances that are known comprise hydrophobic groups and also chemically reactive groups; allergenic and/or irritant properties are presumably due to just these very groups.

Test substances employed in epicutaneous testing are often mixtures of a variety of chemical compounds occurring in particular compositional contexts, e.g. in perfumes. The constituents of the mixtures may be chemically very different in character but nevertheless the invention has been shown to be operable for such mixtures also. The invention is thus potentially useful for Carba mix, colophony (rosin), PPD mix (= black

3

rubber mix), lanolin, mercapto mix, Kain mix, balsam of Peru, oak moss (abs. mousse de chêne), thiuram mix, parabenes, quinoline mix, mix of fragrances etc.

Among test substances comprising a distinct chemical compound to which the stabilizing method of this invention is applicable may be mentioned: isoeugenol, eugenol, geraniol, alpha-amyl cinnamic aldehyde, alpha-hexyl cinnamic aldehyde, cinnamic alcohol, p-phenylenediamine and hydroxycitronellal.

In addition to the test substance stabilized according to above, the test strip according to the invention may also contain test substances formulated by alternative techniques, for example prior art technique.

According to the invention a test strip may on one of its patches contain formaldehyde as the test substance, in the form of a paraformaldehyde inclusion complex, especially cyclodextrin-paraformaldehyde complex.

## Formation of incusion complexes

Inclusion complexes may be produced by means of simple digestion in the presence of a suitable solvent (water). In a preferred embodiment this is achieved in that the test substance and cyclo compound (I) are mixed together and subjected to mechanical working; but alternatively recourse may be had to ordinary complexing methods, for instance by causing the complexes to precipitate in aqueous media.

By varying the molar ratio of test substance to cyclo compound (I) the test substance may be caused to be bound more or less firmly in the complex; consequently the molar ratio controls the amount of test substance to be released when the epicutaneous test is being performed. Therefore, the molar ratio should be chosen such as to enable a correct amount to be released during the test. The optimum ratio will vary according to the test substances, vehicles and testing methods employed. Our studies in connection with this invention have shown that generally the most preferred embodiments are those where the test substance - i.e. the substance which is to penetrate the skin - and the cyclo compound are present in approximately equimolar amounts ($\pm 25$ %), or with an excess of the test substance. The complex obtained may then be formulated in a suitable vehicle, for example in film-forming polymers or in petrolatum, and be employed in accordance with known techniques. See for instance the techniques mentioned in the introductory part of this specification. A very advantageous method is the one described in WO-A-8601994. It involves incorporating the complex in a vehicle which is in the form of a polymeric film, this film being swellable when applied to skin under occlusion. The term "film" here means a thin layer, of less than 0.25 mm thickness. The WO-A-8601994 method shows that film thicknesses of less than 0.1 mm are useful, for instance less than 0.05 mm. According to that method the film is applied as a patch on a test strip.

## Vehicle

The most preferred vehicles are those which, when put to use, are capable of forming films as described in WO-A-8601994. Many of them may be used either according to WO-A-8601994 or in classical patch test procedures, e.g. as salves to be dispensed to Finn Chambers®, Square-test, A1-test etc.

The vehicle may thus preferably be selected from among substances having film-forming properties. The substance usually is a polymer which in combination with a volatile liquid such as e.g. water will form a gel or a coalescent emulsion in which the complex can be distributed homogeneously, in either a dissolved or a crystallized or a micronized or a dispersed form; in the most practical embodiment the polymer will be chosen such that the gel or emulsion when spread out is capable of drying so as to form a coherent film. The vehicle should have hydrophilic properties so that it will be capable of absorbing moisture and swelling to a gel when in use according to WO-A-8601994.

Various properties revealing the hydrophilic character of a polymeric vehicle are inferrable from handbooks and manufacturers' instructions; see for example Encyclopedia of Polymer Science and Technology; Plastics, Rubbers, Resins, Fibers; John Wiley & Sons Inc.; Vol. 6, pp. 778 - 779 (1967). The particular film-forming polymers to be chosen are preferably selected from among those containing multiple polar structures, e.g. carboxyl and/or ester groups, ether groups, such as completely or partially alkylated polysaccharides (e.g. hydroxypropylated, carboxymethylated and methylated cellulose) etc., alcoholic groups such as polysaccharides (e.g. starch) optionally in derivatized forms like the aforementioned alkylated forms, amid group (e.g. polyvinylpyrrolidones). The degree of hydrophilicity will depend on the relative amount of polar groups present in the polymer. Film-forming polymers based on naturally occurring polymers (biopolymers) are as a rule sufficiently hydrophilic.

The exact choice of the vehicle will depend on, inter alia, the complex actually contemplated, and on the subjacent substrate material (which may be aluminum or other material forming the contact surface for the vehicle on the test strip).

In the case of the method according to WO-A-8601994 it has been found essential to choose a film-forming polymer capable of forming a film together with a polar volatile solvent, preferably water, ethanol, methanol etc. or homogeneous mixtures thereof. For cyclodextrin complexes, suitable polymers may be selected from among film-forming polysaccharides and their derivatives. In particular the above-mentioned alkylated forms of polysaccharides, especially of cellulose, have been found to possess and excellent balance of hydrophilicity-hydrophobicity while at the same time being non-irritant to human skin.

During the priority year it has been found that cyclodextrins in connection with certain film-forming polymers, in particular methylated and/or hydroxypropylated celluloses and similar polymers, may result in brittle films. The quality of the film may be improved by replacing this type of polymers with such ones giving more supple and elastic films like polyvinylpyrrolidone (PVP). The most suitable polymer for each cyclocompound/test substance may be found by simple testing.

For the method according to WO-A-8601994 the carrier may consist of a mixture of polymers. In other words, the vehicle is to contain at least one of the polymers contemplated. It is a requirement, however, that the mixture must be capable of giving a film which will swell when applied to skin under occlusion, e.g. in an epicutaneous testing procedure.

Important points to be considered when the vehicle is to be chosen are that the vehicle has to be pharmaceutically acceptable, non-irritant on the skin and inert to the film carrier and that it should not, to any substantial extent, alter the allergenic and/or irritant properties of the test substance contemplated.

Manufacture of the test patch on which the test substance is formulated in a film

There are two important steps in the manufacturing procedure which are of prime importance for the result obtained: (i) The complex has to be distributed uniformly in the film-forming material, and (ii) this latter material has to be spread out in a manner such as to form a film of uniform thickness on a suitable substrate ( = film carrier). For selecting the type of film carrier to be employed see the general directions given in WO-A-8601994.

In accordance with the currently best known method for producing the film the complex is added to a film-forming polymer (vehicle) dissolved or gelled in a volatile liquid. This may be done by dispersing or emulsifying the complex homogeneously in a finely divided state in the gel. As a matter of principle it is also possible to produce the complex in situ in the gelled polymer, the test substance and cyclo compound (preferably cyclodextrin) being in that case added to the gel before the gel is spread out on its substrate. The film carrier is then coated with a uniform layer of the gel which is then allowed to dry, whereupon this material may be cut up into a suitable number of patches, these latter being preferably equal in shape and size (area). The thickness of the film as dried is variable depending on the amount of gel applied. The area of the patches may amount to 0.2 - 4 cm². The amount of test substance in the film per unit area thereof will vary according to the type of allergen (test substance), cyclo compound, molar ratio of cyclo compound to test substance, the vehicle employed etc. Some allergens are more potent than others; the artisan will thus have to carry out some trial and error experimentation in order to find out the suitable effective amount per unit area. The term "effective amount per unit area" means the amount of test substance which when employed in the test will cause an allergic or irritative reaction in most of the sensitized or normal individuals, respectively.

The patches are then placed onto a pressure-sensitive adhesive material which provides a projecting margin of at least about 1 cm all around each patch (the film side is to be placed so as to face away from the adhesive side of the material). The geometrical configuration of the patch is not critical for the purposes of the invention; so in principle strips may be employed as equivalents to other configurations.

To facilitate the testing procedure, it is possible to prearrange a plurality of patches on one common piece of the adhesive material, the individual patches having been provided with different allergens each, and/or with the same allergen in different amounts per unit area. The test strip of the invention contains at least one patch carrying a test substance formulated as an inclusion complex, e.g. cyclodextrin complex. The test strip is to be used for simultaneous testing on the patient against a plurality of allergens and/or respectively, against different amounts of the same allergen per unit area. Such strips may comprise patches corresponding to a standard tray; each strip may contain up to 25, preferably up to 12 patches.

Testing procedure

This procedure is carried out in a known per se manner but with at least one test substance formulated according to the present invention, preferably in the form of a test strip having at least one patch thereon. Thus one or more patches (test strips) are affixed on the patient so that the vehicle (film) will contact the skin in the testing area, whereupon the strip is sealingly pressed against the skin into a fixed position.

The invention will now be illustrated by means of some non-limitative examples.

Example 1

Manufacture of test strip containing a patch with test allergen consisting of a mix of fragrances in a cyclodextrin complex form

5.4 g of a mix of fragrances (equal amounts in grams of geraniol, cinnamic aldehyde, hydroxycitronellal, cinnamic alcohol, eugenol, isoeugenol and amyl cinnamic aldehyde) was added dripwise at room temperature, with stirring, to a mixture of beta-cyclodextrin (30 g) and distilled water (30 g). Stirring was continued until the complex had formed (substantial change in consistency of the mixture. Then followed an addition of 5 g of Klucel® GF (hydroxypropyl cellulose, Hercules Inc., USA) gelled in 100 g of distilled water in accordance with the manufacturer's directions; the whole was stirred vigorously until a homogeneous mixture was obtained. The mixture, of substantially gel consistency, was deposited and spread out as a film of even thickness (about 0.1 mm) on a polyester sheet (Mylar®) which had a thickness of 0.03 mm and had been subjected to corona discharge treatment. After drying, a thin supple coherent film was obtained having a thickness of about 0.01 mm. In a similar manner but without the cyclodextrin component film-coated sheets were prepared on which thus the test allergen was in an uncomplexed state (cp WO-A-8601994). The film-coated sheets were then divided into 1 cm² square patches which were placed on pressure-sensitive adhesive strips (acrylate strips (Lysapor®) from Cederroth, Sweden) with a projecting margin of at least 1 cm. The each test strip was sealingly enclosed in aluminum foil so as to exclude light, air and moisture. The whole was then stored for 1.5 month at +40 °C (complexed test allergen) or for 2 weeks at the same temperature (non-complexed allergen). The amount of test allergen per $cm^2$ was determined initially and then again after storage as aforesaid. The method employed involved extracting a patch of known area with ethanol and then analyzing the resultant extract gas chromatographically on a capillary column (Hewlett-Packard 5700) with the aid of a flame ionization detector. For results see Table 1.

## Table 1

| A. Fragrances + cyclodextrin | Concentration (mg/$cm^2$) | |
|---|---|---|
| | Initially | Storage 1,5 month at + 40°C |
| Geraniol | 0.011 | 0.019 |
| Cinnamic aldehyde | 0.008 | 0.010 |
| Hydroxycitronellal | 0.008 | 0.012 |
| Cinnamic alcohol | 0.013 | 0.018 |
| Eugenol | 0.009 | 0.008 |
| Isoeugenol | 0.004 | 0.003 |
| alfa-amyl cinnamic aldehyde | 0.010 | 0.007 |

| B. Fragrances only | | |
|---|---|---|
| | Initially | Storage 2 weeks at + 40°C |
| Geraniol | 0.013 | < 0.0001 |
| Cinnamic aldehyde | 0.011 | < 0.0001 |
| Hydroxycitronellal | 0.026 | 0.002 |
| Cinnamic alcohol | 0.36 | < 0.0001 |
| Eugenol | 0.024 | < 0.0001 |
| Isoeugenol | 0.031 | 0.0001 |
| alfa-amyl cinnamic aldehyde | 0.045 | 0.0003 |

The results show unambiguously that if the test allergen is in the form of a cyclodextrin complex substantially nothing is destroyed under the conditions set forth. The non-complexed allergen on the other hand is rapidly destroyed. Therefore, if no stabilization were to be carried out it would hardly be possible to guarantee the presence of the intended amount of an unstable test substance in a patch.

Test strips containing patches with fragrances-cyclodextrin complex were then tested clinically on 11 patients suffering from known perfume allergy. Comparative materials employed were corresponding patches without cyclodextrin or the Finn Chambers® method with the test allergen in petrolatum. Strips were placed on the patient's back under occlusion; after 48 hours they were removed, and then after still another 24 hours readings were taken. The results obtained with the patches of the invention showed good agreement with the Finn Chambers® method, but the form of administration as provided by the invention is free from the drawbacks inherent in the Finn Chambers method.

A comparison between uncomplexed and complexed perfume component (both incorporated in Klucel® film) revealed superiority of the complexed form.

Example 2

Manufacture of test patch containing formaldehyde in the form of a paraformaldehyde-cyclodextrin complex

0.5 g of paraformaldehyde was mixed homogeneously with 11.8 g of beta-cyclodextrin, and then 12 g of Klucel® G gel (Klucel®: water = 1 : 6 (w/w)) was added. The mixture was subjected to vigorous kneading until it was homogenous, whereupon it was applied with pressure onto Mylar® sheets to form a 0.1 mm film; this application of the film was carried out in the same manner as in Example 1. The sheets were stored sealed-off in the dark at - 20°C, + 8 °C and + 40 °C for 25 days. The amount of paraformaldehyde was determined as described by Sawicki et al. (Anal. Chem. 33 (1961) pp. 93 -).

Table 2

| Storage conditions | Initial value mg/cm2 | After 25 days mg/cm$^2$ |
|---|---|---|
| - 20 $^{o}$C | 0.07 | 0.07 |
| + 8 $^{o}$C | 0.07 | 0.17 |
| + 40 $^{o}$C | 0.07 | 0.07 |

Sheets that had been produced separately were divided into 1 cm$^2$ square patches. These were placed onto pressure-sensitive adhesive strips of a sticking plaster type (Cederroth acrylate sticking plaster) and were studied clinically in the same manner as in Example 1. The results obtained were consistent with earlier diagnoses.

Example 3

During the priority year mercaptomix, parabenes and quinoline mix have been formulated in accordance with the invention. The method for preparation of the patches has been in accordance with Example 1.

Claims

1. Test strip having at least one patch thereon and intended for use in occlusive epicutaneous testing, **characterized** in that on at least one of said at least one patch the test substance is present as an inclusion complex with a cyclo compound capable of forming inclusion complexes with the test substance.

2. Test strip according to claim 1, **characterized** in that the cyclo compound is a cyclized polysaccharide, especially cyclodextrin having from 6 to 12 alpha-1,4-glucopyranose units.

3. Test strip according to either of claims 1 - 2, **characterized** in that on at least one of said at least one patch the inclusion complex contains formaldehyde as its test substance in the form of paraformaldehyde.

4. Test strip according to either of claims 1 - 2, **characterized** in that on at least one of said at least one inclusion-complex-containing patch the test substance is a mixture of chemical substances such as in perfume, carba mix, lanolin, mercapto mix, colophony, balsam of Peru, oak moss, PPD mix, mix of fragrances, etc.

5. Test strip according to any of claims 1 - 4, **characterized** in that on at least one of said at least one patch the test substance, preferably the one present as an inclusion complex, is formulated in a polymeric film which will absorb moisture excreted from the tested skin area when the patch is applied to skin under occlusion.

6. Test strip according to any of claims 1 - 5, **characterized** in that on at least one of said at least one inclusion-complex-containing patch the molar ratio of test substance to cyclo compound is greater than 75 %.

7. Occlusive epicutaneous testing method **characterized** in that at least one test substance is applied as an inclusion complex with a cyclo compound capable of forming such complexes with the test substance.

8. Method according to claim 7, **characterized** in that the inclusion complex applied is a complex between the test substance and a cyclized polysaccharide, especially a cyclodextrin having from 6 to 12 alpha-1,4-glucopyranose units.

9. Method according to either of claims 7 - 8, **characterized** in that one of the said at least one test substance is formaldehyde in the form of paraformaldehyde.

10. Method according to either of claims 7 - 8, **characterized** in that at least one of the said at least one test substance is a mixture of chemical substances selected from group of test substances consisting of a perfume, carba mix, lanolin, mercapto mix, colophony, balsam of Peru, oak moss, PPD mix, etc.

11. Method according to either of claims 7 - 11, **characterized** in that the molar ratio of test substance to cyclo compound (I) for the inclusion complex is greater than 75 %.

Claims for the following Contracting States : AU, GR, ES

1. Method for the production of a contact allergy test strip by applying to at least one distinct area (patch) on said strip a contact allergy test substance optionally formulated in a vehicle, said strip being intended for use in occlusive epicutaneous testing and said method being **characterized** by applying to at least one patch of the strip the test substance formulated as an inclusion complex between said test substance and a cyclo compound capable of forming an inclusion complex with said test substance.

2. Method according to claim 1, **characterized** by the cyclo compound being a cyclized polysaccharide, especially cyclodextrin having from 6 to 12 alpha-1,4-glucopyranose units.

3. Method according to either of claims 1 - 2, **characterized** by formaldehyde in the form of paraformaldehyde being one of the test substances that are applied to the strip in form of an inclusion complex.

4. Method according to either of claims 1 - 2, **characterized** by a mixture of chemicals substances such as in a perfume, carba mix, lanolin, mercapto mix, colophony, balsam of Peru, oak moss, PPD mix, mix of fragrances, etc. being one of the test substances that are applied to the strip in form of an inclusion complex.

5. Method according to any of claims 1 - 4, **characterized** by the vehicle being a film-forming polymer so selected and so applied that the polymer will create a polymeric film which will absorb moisture excreted from the tested skin area when the strip is in use.

6. Method according to any of claims 1 - 5, **characterized** by the molar ratio of test substance to cyclo compound in at least one of said inclusion-complexes being greater than 75 %.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol.100, no.11, March 12, 1979, page 351, No 91355s, Columbus Ohio (USA); & JP - A - 58 11 3275 (NICHIBAN KK) July 6, 1983 *Abstract* | 1-11 | A 61 K 49/00 A 61 B 10/00 A 61 M 35/00 |
| Y | SE-B-443 914 (T.I. FISCHER) | 1-11 | |
| A | US-A-4 214 592 (P. JACQUET ET AL) | 1-11 | |
| A | US-A-3 703 890 (M.A. SAUNDERS) | 1-11 | |
| A | US-A-3 515 126 (G.S. FREGERT) | 1-11 | TECHNICAL FIELDS SEARCHED (Int. Cl.4) |
| A | US-A-3 699 963 (A. ZAFFARONI) | 1-11 | A 61 K A 61 B A 61 M G 01 N |
| A | US-A-3 734 097 (A. ZAFFARONI) | 1-11 | |
| A | GB-A-1 459 262 (V.P. PIRILA) | 1-11 | |
| | -/- | | |

The present search report has been drawn up for all claims

| Place of search | Date of completion of the search | Examiner |
|---|---|---|
| STOCKHOLM | 25-09-1987 | FORSLUND N. |

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | ACTA PHARMACEUTICA SUECIA, vol. 21, pages 357-366, Stockholm (SWEDEN); M OTAGIRI et al: "comparative study on inclusion complexation of - cyklodextrin and tri-O-methyl- - cyclodextrin with several drugs in aqueous solution" | 1-11 | A 61 K 49/00<br>A 61 B 10/00<br>A 61 M 35/00 |
| | ----- | | |

TECHNICAL FIELDS
SEARCHED (Int. Cl.4)

A 61 K
A 61 B
A 61 M
G 01 N